# EUROPÄISCHE PATENTANMELDUNG

(11) **EP 0 582 146 A2**
(43) Veröffentlichungstag der Anmeldung: **09.02.1994**
(21) Anmeldenummer: 93111782.4
(22) Anmeldetag: 23.07.1993
(51) Int. Cl.: C07D 471/04, A61K 31/435

(54) **Verfahren zur Herstellung von Imidazopyridinen**

(30) Priorität: 05.08.1992 DE 4225835
(71) Anmelder: MERCK PATENT GmbH, D-64271 Darmstadt (DE)
(72) Erfinder: Mederski, Werner, Dr., D-64390 Erzhausen (DE); Bokel, Heinz-Hermann, Dr., D-64291 Darmstadt (DE)

(57) **Zusammenfassung**

Die Erfindung betrifft ein neues Verfahren zur Herstellung von Imidazopyridinen der Formel I 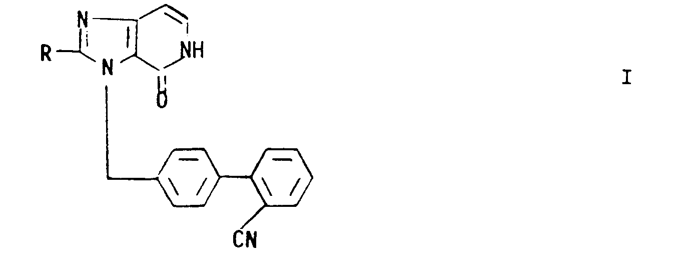
worin
R Alkyl mit 1-6 C-Atomen

bedeutet,
dadurch gekennzeichnet, daß man 3,4-Diamino-2-chlorpyridin (11) mit einem Säureanhydrid der Formel RCO-O-COR' (III), wobei R die angegebene Bedeutung hat und R' = R ist oder ein anderer aliphatischer oder aromatischer Rest sein kann, zu einem 4-Amino-2-chlor-3-R-CO-amino-pyridin (IV) umsetzt, dieses mit 4'-Brommethyl-2-cyan-biphenyl (V) in Gegenwart eines Alkalimetallalkoholats in einem inerten Lösungsmittel in ein 4-Amino-2-chlor-3-R-CO[N-(2'-cyanbiphenylyl-4-methyl)- amino]-pyridin (VI) überführt und dieses mit einer starken Säure behandelt, wobei als Zwischenprodukt ein 2-R-4-chlor-3-(2'-cyanbiphenylyl-4-methyl)-3H-imidazo[4,5-c]pyridin (VII) entsteht.

## Beschreibung

Gegenstand der Erfindung ist ein neues Verfahren zur Herstellung von Imidazopyridinen der Formel I 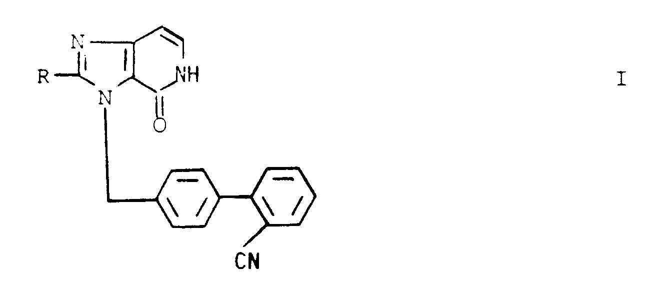
worin
R Alkyl mit 1-6 C-Atomen

bedeutet,
dadurch gekennzeichnet, daß man 3,4-Diamino-2-chlorpyridin (11) mit einem Säureanhydrid der Formel RCO-O-COR' (III), wobei R die angegebene Bedeutung hat und R' = R ist oder ein anderer aliphatischer oder aromatischer Rest sein kann, zu einem 4-Amino-2-chlor-3-R-CO-amino-pyridin (IV) umsetzt, dieses mit 4'-Brommethyl-2-cyan-biphenyl (V) in Gegenwart eines Alkalimetallalkoholats in einem inerten Lösungsmittel in ein 4-Amino-2-chlor-3-R-CO-[N-(2'-cyanbiphenylyl-4-methyl)- amino]-pyridin (VI) überführt und dieses mit einer starken Säure behandelt, wobei als Zwischenprodukt ein 2-R-4-chlor-3-(2'-cyanbiphenylyl-4-methyl)-3H-imidazo[4,5-c]pyridin (VII) entsteht.

Verbindungen der Formel I hemmen die Wirkung des Angiotensins II und können dementsprechend als Arzneimittelwirkstoffe, insbesondere zur Senkung des Blutdrucks, verwendet werden. Sie eignen sich ferner als Zwischenprodukte zur Herstellung anderer Arzneimittelwirkstoffe.

Der Rest R ist vorzugsweise geradkettig und bedeutet vorzugsweise Butyl, ferner Propyl, aber auch Methyl, Ethyl, Pentyl, Hexyl, Isopropyl, Isobutyl, sek.-Butyl, tert.-Butyl, 1-, 2- oder 3-Methylbutyl, 1,1-, 1,2-oder 2,2-Dimethylpropyl, 1-, 2-, 3- oder 4-Methylpentyl, 1- oder 2-Ethylbutyl, 1,1-, 1,2-, 1,3-, 2,2-, 2,3- oder 3,3-Dimethylbutyl, 1,1,2- oder 1,2,2-Trimethylpropyl.

Der Rest R' bedeutet vorzugsweise R; in diesem Fall handelt es sich beilll um ein Säureanhydrid einer (einzigen) Säure. Der Rest R' kann aber auch einen anderen aliphatischen oder aromatischen Rest bedeuten; in diesem Fall istlll ein "gemischtes" Säureanhydrid. Vorzugsweise bedeutet R' in diesem Fall einen aliphatischen oder aromatischen Kohlenwasserstoffrest mit jeweils bis zu 10 C-Atomen, z.B. Alkyl, insbesondere verzweigtes Alkyl wie tert.-Butyl, oder unsubstituiertes oder durch 1-5 niedere Alkylgruppen, insbesondere Methyl, substituiertes Phenyl wie 3,5-Dimethylphenyl oder 2,4,6-Trimethylphenyl.

Die Umwandlung eines 3,4-Diaminopyridins in ein 2-R-lmidazo[4,5-c]pyridinerfolgt üblicherweise durch Reaktion mit einer Säure der Formel R-COOH in Gegenwart von Polyphosphorsäure oder POCI₃ bei relativ hohen Temperaturen. Setzt man z.B. 11 mit Valeriansäure in Gegenwart von Polyphosphorsäure bei 100-180 _{°} C um, so erhält man - unter gleichzeitiger Hydrolyse des C1-Atoms - 2-Butyl-4,5-dihydro-4-oxo-1 (oder3)H-imidazo[4,5-c]pyridin als Hauptprodukt. Dieses hat den Nachteil, daß bei der "Alkylierung" mit V ein Gemisch von Produkten entsteht.

Der Erfindung lag die Aufgabe zugrunde, diesen Nachteil der üblichen Verfahrensweise zu vermeiden und ein verfahren aufzufinden, bei dem - auf einer beliebigen Stufe - die "Alkylierung" mit V selektiv an der gewünschten Stelle erfolgt. Diese Aufgabe wurde durch das beanspruchte Verfahren gelöst.

Setzt man nämlich II mit einem Säureanhydrid III um, so erhält man in hoher Ausbeute selektiv IV. Dabei arbeitet man bevorzugt unter relativ milden Bedingungen in Gegenwart eines inerten Lösungsmittels bei Temperaturen zwischen 0 und 100°, insbesondere zwischen 10 und 50 °C, wobei man die berechnete Menge an 111, also keinen Überschuß, verwendet. Als Lösungsmittel eignet sich z.B. ein Ether wie Tetrahydrofuran (THF) oder Dioxan.

Die Umsetzung von IV mit V (bekannt aus EP 253 310, Beispiel 89) gelingt erfindungsgemäß in einem inerten Lösungsmittel, vorzugsweise einem polaren Lösungsmittel, z.B. einem Amid wie Dimethylformamid oder einem Lactam wie N-Methylpyrrolidon (NMP), in Gegenwart einer Base, vorzugsweise eines Alkalimetallalkoholats wie Kalium-tert.-butylat, aber auch Natriumoder Kalium-methylat oder -ethylat.

Bevorzugt arbeitet man bei Temperaturen zwischen etwa 0 und 50°, insbesondere zwischen 10 und 20 °C, wobei man zunächst IV mit Hilfe des Alkoholats deprotoniert und anschließend eine Lösung von V zutropft. Unter diesen Bedingungen erhält man überraschenderweise selektiv VI als Hauptprodukt und das daraus durch Wasserabspaltung entstehende VII als Nebenprodukt.

Das erhaltene VI (oder VII oder ein Gemisch aus VI und VII) wird anschließend mit einer starken Säure behandelt, vorzugsweise einer starken Mineralsäure wie Salzsäure oder Schwefelsäure und vorzugsweise in Gegenwart eines zusätzlichen inerten Lösungsmittels oder Lösungsmittelgemischs, z.B. Wasser/ NMP, zweckmäßig bei Temperaturen zwischen 0 und 110 °C, vorzugsweise 100 und 110 °C. Dabei wird VI zu VII cyclisiert und außerdem das Chloratom hydrolytisch abgespalten.

Es ist auch möglich, mehrere Stufen in einem "Eintopfverfahren" zusammenzufassen, wobei man Zwischenprodukte nicht isoliert. Insbesondere gelingt es, die Umsetzung von IV mit V zu VI bzw. VII und die anschließende Abspaltung des Chloratoms in einer Stufe durchzuführen, wobei man VI und VII nicht isoliert.

### Beispiel 1

(a) Man tropft 186 g Valeriansäureanhydrid zu einer Lösung von 143,5 g 3,4-Diamino-2-chlorpyridin in 1350 ml THF und rührt 16 Std. bei 20 °C. Es werden 1 I gesättigte NaHC0₃-Lösung und 340 ml gesättigte Na₂C0₃-Lösung zugegeben. Man filtriert, extrahiert das Filtrat mit Ethylacetat, trocknet über Na₂ S0₄, dampft ein und erhält 4-Amino-2-chlor-3-valeramido-pyridin (IVa), F. 163 °C.
(b) Eine Lösung von 36,8 g K-tert.-Butylat in 100 ml NMP wird unter Rühren bei 10-15 °C zu einer Lösung von 64,9 g IVa in 300 ml NMP zugetropft. Nach weiterem halbstündigem Rühren tropft man 85,2 g V in 300 ml NMP bei 10-15 °C hinzu. Nach weiterem 16stündigem Rühren arbeitet man mit Ethylacetat und gesättigter NaCI-Lösung auf. Kristallisation des Rohprodukts aus Ethylacetat/tert.-Butylmethyl-ether liefert 40 g 4-Amino-2-chlor-3-N-(2'-cyanbiphenylyl-4-methyl) -valeramido-pyridin (Vla), F. 144 °C. Aus der Mutterlauge lassen sich durch Umkristallisation noch 13,7 g 2-Butyl-4-chlor-3-(2'- cyanbiphenylyl-4-methyl)-3H-imidazo[4,5-c]pyridin (Vlla) gewinnen, F. 133,5 °C.
(c) Ein Gemisch von 43,1 g Vla, 36,2 g Vlla, 2000 ml 15%iger Salzsäure und 1200 ml NMP wird bei 105 _{°} C 48 Std. gerührt. Man kühlt ab, stellt mit Natronlauge auf pH 9, extrahiert mit Ethylacetat, filtriert, wäscht mit Wasser, trocknet über Na₂S0₄ und erhält 64 g 2-Butyl-3-(2'-cyanbiphenyly14-methyl)-4,5-dihydro-4-oxo-3H-imidazo[4,5-c]pyridin (la), F. 165 °C.

### Beispiel 2

Man löst 227,7 g IVa bei 5 ° C unter N₂ in 1000 ml NMP, tropft zu dieser Lösung bei 5-10 ° C unter Rühren eine Lösung von 129,2 g K-tert.-Butylat in 400 ml NMP, rührt 1 Std. und tropft dann bei 5-10 _{°} C eine Lösung von 299,4 g V in 750 ml NMP hinzu. Nach 5 Std. Rühren bei 20 _{°} C gibt man 3100 ml 18%ige Salzsäure hinzu und erwärmt 40 Std. auf 105 °C. Es wird auf 80 °C abgekühlt, dann werden 3700 ml 16%ige Natronlauge zugetropft. Man kühlt ab, filtriert, wäscht mit Wasser, kristallisiert aus Ethanol/Wasser 1:1 um und erhält 316 g la, F. 165 °C.

## Patentansprüche

1. Verfahren zur Herstellung von Imidazopyridinen der Formel I 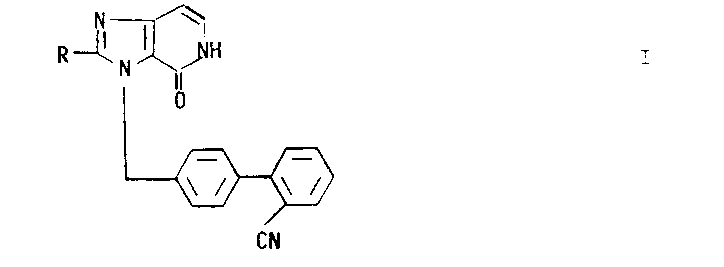
worin
R Alkyl mit 1-6 C-Atomen
bedeutet,
dadurch gekennzeichnet, daß man 3,4-Diamino-2-chlorpyridin (11) mit einem Säureanhydrid der Formel RCO-O-COR' (III), wobei R die angegebene Bedeutung hat und R' = R ist oder ein anderer aliphatischer oder aromatischer Rest sein kann, zu einem 4-Amino-2-chlor-3-R-CO-amino-pyridin (IV) umsetzt, dieses mit 4'-Brommethyl-2-cyan-biphenyl (V) in Gegenwart eines Alkalimetallalkoholats in einem inerten Lösungsmittel in ein 4-Amino-2-chlor-3-RCO-[N-(2'cyanbiphenylyl-4-methyl)- amino]-pyridin (VI) überführt und dieses mit einer starken Säure behandelt, wobei als Zwischenprodukt ein 2-R-4-chlor-3-(2'-cyanbiphenylyl-4-methyl)-3H-imidazo[4,5-c]pyridin (VII) entsteht.
